# EUROPÄISCHE PATENTSCHRIFT

(11) **EP 1 049 455 B1**
(45) Veröffentlichungstag und Bekanntmachung des Hinweises auf die Patenterteilung: **16.08.2001**
(21) Anmeldenummer: 99908763.8
(22) Anmeldetag: 28.01.1999
(51) Int. Cl.: A61K 7/48, A61K 7/06

(54) **KOSMETISCHES PRODUKT AUF BASIS VON ARTEMIA SALINA - EXTRAKTEN ZUR REGENERIERUNG UND STIMULIERUNG DER HAUTZELLEN**
COSMETIC PRODUCT BASED ON ARTEMIA SALINA EXTRACTS FOR REGENERATING AND STIMULATING SKIN CELLS
PRODUIT COSMETIQUE A BASE D'EXTRAITS D'ARTEMIA SALINA POUR LA REGENERATION ET LA STIMULATION DES CELLULES DE LA PEAU

(30) Priorität: 29.01.1998 DE 19804837
(43) Veröffentlichungstag der Anmeldung: 08.11.2000
(73) Patentinhaber: Coty B.V., 2031 CC Haarlem (NL)
(72) Erfinder: ZASTROW, Leonhard, MC-98000 Monte-Carlo (MC); DOMLOGE, Nouha, F-06190 Roquebrunne-Cap Martin (FR); GOLZ-BERNER, Karin, MC-98000 Monte-Carlo (MC)
(74) Vertreter: Walter, Wolf-Jürgen
(86) Internationale Anmeldenummer: DE9900283
(87) Internationale Veröffentlichungsnummer: WO9938483

(56) Entgegenhaltungen:
- FR-A- 1 536 017
- FR-A- 2 053 399
- FR-A- 2 279 382

## Beschreibung

Die Erfindung betrifft ein kosmetisches Produkt zur Regenerierung und Immunsystemstimulierung der Hautzellen.

Aus einigen Veröffentlichungen, z.B. aus Parfümerie und Kosmetik Nr. 12/1995, S. 776-779 ist bekannt, daß eine Reihe spezieller pflanzlicher Rohstoffe aus dem Meer in kosmetischen Präparaten eingesetzt werden, um z.B. freie Radikale zu binden (Superphyto-D), um IR-Schutz zu geben (Phycocorail), um die Hautalterung zu verbessern (Aosaine) und um feuchtigkeitshaltende Wirkungen auszuüben (Codium tomentosum).

Der Erfindung liegt die Aufgabe zugrunde, andere maritime Bestandteile in kosmetische Formulierungen zu integrieren und dabei vorteilhafte Wirkungen zu erzielen.

Erfindungsgemäß besteht ein kosmetisches Produkt zur Regenerierung und Immunsystemstimulierung der Hautzellen auf Basis maritimer Bestandteile aus 0,01 bis 50 Gew-%, bezogen auf die Gesamtmasse, einer Wirkstoffkombination aus
a) einem Produkt eines enzymatischen Extraktionsprozesses des maritimen Planktons Artemia salina, wobei das Extraktionsprodukt aus phosphorylierten Nucleotiden mit der Hauptkomponente Diguanosin-tetraphosphat besteht;
b) D-myo-Inosit-1,4,5-triphosphat;
c) Glycan;
wobei das Verhältnis a:b:c im Bereich 1 : 0,1-50 : 0,1-30 liegt; und einem Gehalt an weiteren kosmetischen Wirk- und Trägerstoffen von 99,99 bis 50 Gew-%.

Vorzugsweise liegt das Verhältnis a:b:c im Bereich 1 : 0,5-10 : 0,5-8.

Ein bevorzugter Bereich für die Wirkstoffkombination liegt bei 0,1 bis 30 Gew-%.

Überraschenderweise werden bei der enzymatischen Extraktion des maritimen Planktons Artemia salina phosphorylierte Nucleotide mit der Hauptkomponente Diguanosin-tetraphoshat (im folgenden GP4G) freigesetzt. "Hauptkomponente" bedeutet, daß mehr als 5 Gew-%, vorzugsweise mehr als 10 Gew-% GP4G in dem Extraktionsprodukt vorhanden sind. Von verschiedenen Guanosinphosphaten sind eine Vielzahl von hormon- und transmitterinduzierten Prozessen bekannt, wobei dem hier interessierenden GP4G keine kosmetisch klar erkennbare Rolle zugeordnet werden kann.

Von Inosit-1,4,5-triphosphat (im folgenden IP₃) ist bekannt, daß es u.a. als intrazelluäre Botensubstanz (second messenger) für bestimmte Neurotransmitter, Hormone und Wachstumsfaktoren die Ausschüttung von Calcium-Ionen aus intrazellulären Reservois in das Cytoplasma bewirkt.

Glycan ist eine pflanzliche Polydextrose, bestehend aus Glucose, Fructose und Dextrin, die biotechnologisch transformiert worden ist. Glycan ist ein Energielieferant zur Stimulierung des Zellmetabolismus.

überraschend bei der erfindungsgemäßen Zusammensetzung war, daß eine von der Gesamtkombination für den Fachmann nicht zu erwartende komplexe Wirkungsweise des Produktes eintritt. Die Wirkung der erfindungsgemäßen Zusammensetzung besteht insbesondere darin, daß eine Stimulierung der Keratinsynthese erfolgt, wodurch die Hautbarriere gegen Umwelteinflüsse auf natürliche Weise verstärkt wird. Zugleich tritt ein Stimulierungseffekt auf das epidermale Immunsystem auf. Weiterhin wird durch eine verstärkte Hautregenerierung ein Reparatureffekt bewirkt, der über längere Zeiträume die Alterung der Haut zurückdrängen kann. Ein weiterer Effekt besteht in der Produktion eines höheren Kollagenanteiles und anderer Hautproteine. In vitro wird deutlich die Hautmorphologie verbessert.

Insgesamt wird durch die neue Zusammensetzung ein Produkt mit Synergie-Effekt bereitgestellt, das aggressiven Umwelteinflüssen durch natürliche Verstärkung des Immunsystems der Haut Widerstand entgegensetzt, die Hautregenerierung stimuliert und zugleich Schutz gegen UV-Licht durch die verbesserte Keratin-Barriere bietet.

Die Verwendung des erfindungsgemäßen kosmetischen Präparates kann z.B. erfolgen in Sonnencremes, Sonnengelen, After-sun-Produkten, Tagescremes, Nachtcremes, Masken, Körperlotionen, Körpergelen, Reinigungsmilch, Körperpuder, Augenkosmetik, Haarmasken, Haarspülungen, Haarshampoos, Duschgelen, Duschölen, Badeölen sowie in der dekorativen Kosmetik bei Make up's, Lippenstiften, Nagellacken, Mascaras usw. Die Herstellung derartiger Produkte erfolgt auf eine Weise, wie sie dem Fachmann auf diesem Gebiet bekannt ist.

Vorteilhaft für das erfindungsgemäße Produkt ist der Einschluß der Wirkstoffkombination in Liposome, um ein langsames Eindringen in die oberen Hautschichten zu erreichen.

Liposome sind vollständig geschlossene Lipid-Bilayer-Membranen, die ein wäßriges Volumen eingeschlossen enthalten. Liposome können unilamellare Vesikel sein (die eine Einzelmembran-Bilayer besitzen) oder multilamellare Vesikel (Onion-ähnliche Strukturen, gekennzeichnet durch Mehrfachmembran-Bilayer, von denen jede von der nächsten durch eine wäßrige Schicht getrennt ist). Die Bilayer besteht aus zwei Lipid-Monolayern, die einen hydrophoben "Schwanz"-Bereich und einen hydrophilen "Kopf"-Bereich haben. Die struktur der Membran-Bilayer ist so, daß die hydrophoben (unpolaren) "Schwänze" der Lipidmonolayer sich in Richtung des Zentrums der Bilayer orientieren, während sich die hydrophilen "Köpfe" in Richtung der wäßrigen Phase orientieren.

Die Herstellung von Liposomen, aus gesättigten und ungesättigten Lipiden, ist in sehr vielen Patenten beschrieben worden, ebenso deren Einsatz als Transportsystem. Die Einarbeitung der erfindungsgemäßen Wirkstoffkombination kann auf übliche Weise erfolgen.

Vorteilhaft für die erfindungsgemäßen Zubereitungen kann es auch sein, daß die Zubereitung die Wirkstoffkombination eingelagert in asymmetrische lamellare Aggregate enthält, wobei diese Aggregate aus Phospholipiden und mit Sauerstoff beladenem Fluorcarbon oder Fluorcarbongemisch bestehen, deren Gehalt an Fluorcarbon im Bereich von 0,2 bis 100 % Gewicht/Volumen liegt, wobei das Phospholipid einen Phosphatidylcholingehalt von mehr als 30 bis 99 Gewichts-% hat, und wobei diese Aggregate eine Hautpenetrierung in Abhängigkeit von der kritischen Löslichkeitstemperatur der Fluorcarbone besitzen (WO94/00098).

Diese Aggregate können auch zusätzlich allein nur mit Sauerstoff beladen in der kosmetischen Zubereitung vorliegen.

Diese Aggregate sind Sauerstoffträger und ermöglichen ein Penetrieren des Sauerstoffs in die Haut und damit eine bessere Versorgung der Haut mit Sauerstoff. Die Herstellung dieser Aggregate erfolgt durch Hochdruckhomogenisierung von Phospholipiden, wie Sojalecithin und Eilecithin oder synthetischen Phospholipiden oder teilhydrierten Phospholipiden, die einen Phosphatidylcholingehalt von mehr als 30 Gew.-% bis 99 Gew.-% haben, mit perfluorierten oder hochfluorierten Kohlenstoffverbindungen oder Gemischen davon, die in der Lage sind, Gase, wie Sauerstoff und Kohlendioxid zu transportieren. Darin können neben Phosphatidylcholin auch Lysolecithine im Konzentrationsbereich von 0,1 bis 10 Gew.-% und/oder geladene Phospholipide wie Phosphatidylethanolamin, n-Acetylphosphatidylethanolamin oder Phosphatidsäure im Konzentrationsbereich 0,1 bis 30 Gew.-% vorhanden sein.

Der Anteil der mit der erfindungsgemäßen Wirkstoffkombination beladenen Aggregate oder im Gemisch damit vorliegenden Aggregate kann im Bereich von 0,1 bis 30 Gew.-% liegen, bezogen auf die Gesamtzubereitung, und liegt vorteilhaft im Bereich von 1 bis 20 Gew.-%.

Als weiteren Wirkstoff kann das Präparat vorteilhaft Kaolin gemäß WO96/17588 enthalten, der mit sphärischen TiO₂- oder SiO₂-Teilchen mit einer Teilchengröße <5 *µ*m modifiziert ist, wobei die sphärischen Teilchen einen Anteil an der Kaolinmischung von 0,5 bis 10 Gew-% haben. Das Präparat erhält dadurch ein sehr weiches Hautgefühl und eine zusätzliche entzündungswidrige Wirksamkeit.

Der modifizierte Kaolin kann einen Anteil von 0,1 bis 15 Gew-% haben, bezogen auf die Gesamtmenge des Produktes.

Das erfindungsgemäße Präparat enthält weiterhin kosmetische Hilfs- und Trägerstoffe, wie sie üblicherweise in solchen Zubereitungen verwendet werden, z.B. Wasser, Konservierungsmittel, Vitamine, Farbstoffe, Pigmente mit färbender Wirkung, Radikalfänger, Verdickungsmittel, weichmachende Substanzen, feuchthaltende Substanzen, Duftstoffe, Alkohole, Polyole, Elektrolyte, Gelbildner, polare und unpolare öle, Polymere, Copolymere, Emulgatoren, Wachse, Stabilisatoren.

Wenn das erfindungsgemäße Produkt zusätzlich Erweichungsmittel enthält, können als Erweichungsmittel normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Oleylalkohol, Isopropyllaurat, Decyloleat, Octadecan-2-ol, Isocetylalkohol, Cetylpalmitat, Siliconöle wie Dimethylpolysiloxan, Isopropylmyristat, Isopropylpalmitat, Polyethylenglycol, Lanolin, Kakaobutter, pflanzliche öle wie Maisöl, Baumwollsamenöl, Olivenöl, mineralische Öle, Butylmyristat, Palmitinsäure usw.

Die erfindungsgemäße Zusammensetzung kann vorteilhaft auch Antioxidationsmittel enthalten. Zu Antioxidationsmitteln gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, ß-Carotin; Harnsäure und Derivate davon; α-Hydroxysäuren wie Citronensäure, Milchsäure, Apfelsäure; Stilbene und deren Derivate usw.

Zusätze von Vitamin A bzw. Vitamin A-palmitat (Retinol) und Vitamin E sind besonders bevorzugt.

Es ist weiterhin vorteilhaft, den erfindungsgemäßen Zusammensetzungen entsprechende wasser- und/oder öllösliche UVA-oder UVB-Filter oder beide zuzusetzen. Zu vorteilhaften öllöslichen UVB-Filtern gehören 4-Aminobenzoesäure-Derivate wie der 4-(Diemethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Wasserlösliche UVB-Filter sind z.B. Sulfonsäurederivate von Benzophenon oder von 3-Benzylidencampher oder Salze wie das Na- oder K-Salz der 2-Phenmylbenzimidazol-5-sulfonsäure.

Zu UVA-Filtern gehören Dibenzoylmethan-Derivate wie 1-Phenyl-4-(4'-isopropylphenyl)propan-1,3-dion.

Bevorzugt als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch eingesetzt werden können.

Besonders bevorzugt als anorganische Pigmente sind agglomerierte Substrate von TiO₂ und/oder ZnO (in den Beispielen als TiO_{2 aggl} bzw ZnO_{aggl} bezeichnet), die einen Gehalt an sphärischen und porösen SiO₂-Teilchen aufweisen, wobei die SiO₂-Teilchen eine Teilchengröße im Bereich von 0,05 *µ*m bis 1,5 *µ*m haben, und neben den SiO₂-Teilchen andere anorganische teilchenförmige Stoffe mit sphärischer Struktur vorliegen, wobei die sphärischen SiO₂-Teilchen mit den anderen anorganischen Stoffen definierte Agglomerate mit einer Teilchengröße im Bereich von 0,06 *µ*m bis 5 *µ*m bilden. Dabei sind besonders vorteilhaft einzusetzende SiO₂-Teilchen hochmonodisperse, unporöse, sphärische SiO₂-Teilchen gemäß DE 3616133, die durch hydrolytische Polykondensation von Tetraalkoxysilan in wäßrigalkoholisch-ammoniakalischen Medium erzeugt werden, wobei ein Sol von Primärteilchen erzeugt wird und anschließend durch ein kontinuierliches, nach Maßgabe des Abreagierens kontrolliertes Zudosieren von Tetraalkoxysilan die erhaltenen SiO₂-Teilchen auf die gewünschte Teilchengröße von etwa 0,05 bis 10 *µ*m bringt.

Das erfindungsgemäße Produkt kann als O/W- , W/O-, W/O/W-, Silicon/W/O-Emulsion oder als Gel vorliegen. Für O/W- und W/O- Emulsionen eignen sich sehr gut bestimmte polare pflanzliche öle. Dazu gehören Rizinusöl, Weizenkeimöl, Traubenkernöl, Nachtkerzenöl, Jojobaöl, Kukuinußöl, Distelöl und weitere öle mit wenigstens 1,5 Gew-% Linolsäureglyceriden. Mehrfachemulsionen und deren Komponenten sind ebenfalls bekannt; es können auch mehrphasige Emulsionen wie in der WO96/41613 beschrieben hergestellt werden.

Im Produkt können auch biogene Wirkstoffe enthalten sein, wie z.B. Pflanzenextrakte, Enzyme und Vitaminkomplexe.

Die Erfindung soll nachstehend durch Beispiele näher erläutert werden. Alle Angaben erfolgen in Gewichtsprozent, sofern nichts anderes angegeben ist.

### Beispiel 1 Gesichtsmaske I

| | |
|---|---|
| Carbomer | 1,5 |
| Triethanolamin | 1,0 |
| Isopropanol | 10,0 |
| Propylengycol | 3,0 |
| Dow Corning Fluid 200 | 1,0 |
| Kaolin gemäß WO96/17588 | 10,0 |
| Konservierungsmittel | 0,5 |
| Komplex GL | 40,0 |
| Retinol Vitamin A-palmitat | 0,5 |
| deionisiertes Wasser | ad 100 |

Unter "Komplex GL" wird die erfindungsgemäße Wirkstoffkombination verstanden, bestehend aus etwa gleichen Teilen an GP4G, IP₃ und Glycan in Wasser. Zur Herstellung bestimmter Ausgangskonzentrationen, z.B. einer Konzentration von 2% werden die entsprechenden Mengen mit Wasser bei Raumtemperatur unter einem Vakuum von bis zu 100 Millibar langsam (mit ca. 300 U/min ca. 2-3 Stunden gerührt.

In dem Wasser wird Carbomer verrührt und auf etwa 60 bis 65 °C erwärmt. Nach der Neutralisierung mit Triethanolamin und Abkühlung auf etwa 40 °C erfolgt die Zugabe der weiteren Komponenten, und es wird längere Zeit gerührt. Bei etwa 30 bis 35 °C wird abschließend der Komplex GL eingerührt.
GP4G und Glycan sind Handelsprodukte der Laboratoires Seporga, Sophia-Antipolis, Frankreich.

### Beispiel 2 Gesichtsmaske II

| | |
|---|---|
| Carbomer | 1,5 |
| Triethanolamin | 1,0 |
| Isopropanol | 10,0 |
| Propylengycol | 3,0 |
| Dow Corning Fluid 200 | 1,0 |
| Kaolin gemäß WO96/17588 | 10,0% |
| Konservierungsmittel | 0,5 |
| Komplex GL | 40,0 |
| Liposome GL | 10,0 |
| Retinol Vitamin A-palmitat | 1,0 |
| deionisiertes Wasser | ad 100 |

Unter "Komplex GL" wird die erfindungsgemäße Wirkstoffkombination verstanden im Verhältnis a:b:c = 1:1,2:2,3. Diese Wirkstoffkombination wird auch in Liposome eingetragen. Die Liposome werden hergestellt aus 10 % Lecithin, in den der Komplex GL unter starkem Rühren eingebracht wird. Danach erfolgt die Zugabe von Wasser, 3 % Glycerin und 7 % Ethanol, wonach wiederum stark gerührt wird.

Es wird bei der Herstellung der Maske II wie im Beispiel 1 verfahren. Die Liposome GL werden am Schluß unter vorsichtigem Rühren hinzugegeben.

### Beispiel 3 Mascara

| **Phase A** | |
|---|---|
| Magnabrite HV | 0,3 |
| Wasser (bezogen auf Gesamtgemisch) | ad 100 |
| Cellulose gum | 0,5 |
| Farbpigmente (je nach Farbe) ca. | 0,3 |

| **Phase B** | |
|---|---|
| Carnaubawachs | 1,0 |
| Bienenwachs | 9,0 |
| Emerwachs | 8,5 |

| **Phase C** | |
|---|---|
| Konservierungsmittel | 0,5 |
| Morpholine | 0,4 |

| **Phase D** | |
|---|---|
| Komplex GL | 0,01 |
| Retinol Vitamin A-palmitat | 2,0 |

Unter "Komplex GL" wird die erfindungsgemäße Wirkstoffkombination verstanden im Verhältnis a:b:c = 1:2,5:1,5.

Die Phasen A, B und C werden separat auf 85 °C erwärmt, unter Rühren zusammengegeben und dann gut homogenisiert. Nach dem Abkühlen wird die Phase D bei 30 bis 35 °C hinzugegeben.

### Beispiel 4 Creme für Tag und Nacht

| **Phase A** | |
|---|---|
| weiße Vaseline | 10,0 |
| Paraffinöl | 5,0 |
| Stearinsäure | 3,0 |
| Carbomer | 0,5 |
| Jojobaöl | 3,0 |

| **Phase B** | |
|---|---|
| Wasser (bezogen auf Gesamtgemisch) | ad 100 |
| Glycerin | 5,0 |

| **Phase C** | |
|---|---|
| Triethanolamin | 0,5 |

| **Phase D** | |
|---|---|
| Vitamin A-palmitat | 0,1 |
| Kaolin gemäß WO96/17588 | 2,3 |
| Konservierungsmittel | 0,5 |
| Parfüm | ca. 0,2 |
| Komplex GL | 10,0 |

Unter "Komplex GL" wird die erfindungsgemäße Wirkstoffkombination verstanden im Verhältnis a:b:c = 1:1,3:1,6.

Die Phasen A und B werden separat auf 70-75 °C unter Rühren erwärmt und dann zusammengegeben. Danach erfolgt die homogene Zugabe der Phase C, und es wird weiter gerührt. nach Abkühlen unter Rühren auf ca 30-35 °C wird Phase D zugegeben.

### Beispiel 5 Nachtcreme

Die Zusammensetzung entsprach der von Beispiel 4. Es wird jedoch der Komplex GL ersetzt durch 3,0 % in Liposomen verkapseltem Komplex GL. Dieser enthält die erfindungsgemäße Wirkstoffkombination im Verhältnis a:b:c = 1:10:0,1.
Die Arbeitsweise entsprach der des Beispiels 4.

### Beispiel 6 Haarmaske

| **Phase A** | |
|---|---|
| Carbomer | 0,6 |
| Wasser (bezogen auf Gesamtgemisch) | ad 100 |

| **Phase B** | |
|---|---|
| Jojobaöl | 3,0 |
| Paraffinöl | 5,0 |
| Bienenwachs | 3,2 |

| **Phase C** | |
|---|---|
| Triethanolamin | 0,6 |

| **Phase D** | |
|---|---|
| Keratin | 2,0 |
| Konservierungsmittel | 0,5 |
| Parfümöl | 0,8 |

| **Phase E** | |
|---|---|
| Komplex GL | 20,0 |
| Liposome mit Komplex GL | 10,0 |

Unter "Komplex GL" wird die erfindungsgemäße Wirkstoffkombination verstanden im Verhältnis a:b:c = 1:8:2. In den Liposomen betrug das Verhältnis a:b:c = 1:10:0,1.

Die Phasen A und B werden auf 60 bis 65 °C erwärmt und zusammengegeben. Danach erfolgt die Zugabe der Phase C unter Rühren. Nach dem Abkühlen auf 40-45 °C wird unter Rühren die Phase D hinzugegeben. Nach weiterem Abkühlen auf 30-35 °C wird Phase E zugegeben.

### Beispiel 7 Sonnenmilch

| **Phase A** | |
|---|---|
| Cetylalkohol | 5,0 |
| Stearinsäure | 4,0 |

| **Phase B** | |
|---|---|
| Wasser (bezogen auf Gesamtgemisch) | ad 100 |
| Glycerin | 2,0 |

| **Phase C** | |
|---|---|
| Triethanolamin | 0,9 |

| **Phase D** | |
|---|---|
| TiO_{2 aggl} | 5,0 |
| ZnO_{aggl} | 2,0 |
| SiO₂ | 0,5 |
| Kaolin gem. WO96/17588 | 0,3 |
| Parsol MCX | 3,0 |

| **Phase E** | |
|---|---|
| Konservierungsmittel | 0,5 |
| Parfümöl | 0,2 |
| Komplex GL | 3,0 |
| Liposome mit Komplex GL | 5,0 |

Das Verhältnis der Wirkstoffkombination entsprach der von Beispiel 6. Die Verarbeitung erfolgte wie im Beispiel 4.

### Beispiel 8 Shampoo/Duschgel

| | |
|---|---|
| Texapon | 25,0 |
| Amphotensid | 5,0 |
| Wasser | ad 100 |
| Allantoin | 0,2 |
| Konservierungsmittel | 0,5 |
| Komplex GL | 0,5 |
| Parfüm | 0,3 |

Unter "Komplex GL" wird die erfindungsgemäße Wirkstoffkombination verstanden im Verhältnis a:b:c = 1:0,1:1,5.

## Patentansprüche

1. Kosmetisches Produkt zur Regenerierung und Stimulierung der Hautzellen auf Basis maritimer Bestandteile, gekennzeichnet durch 0,01 bis 50 Gew-%, bezogen auf die Gesamtmasse, einer Wirkstoffkombination aus
a) einem Produkt eines enzymatischen Extraktionsprozesses des maritimen Planktons Artemia salina, wobei das Extraktionsprodukt aus phosphorylierten Nucleotiden mit der Hauptkomponente Diguanosin-tetraphosphat besteht;
b) D-myo-Inosit-1,4,5-triphosphat;
c) Glycan;
wobei das Verhältnis a:b:c im Bereich 1 : 0,1-50 : 0,1-30 liegt;
und einem Gehalt an weiteren kosmetischen Wirk- und Trägerstoffen von 99,99 bis 50 Gew-%.

2. Produkt nach Anspruch 1, dadurch gekennzeichnet, daß das Verhältnis a:b:c im Bereich 1 : 0,5-10 : 0,5-8 liegt.

3. Produkt nach Anspruch 1, dadurch gekennzeichnet, daß ein Teil der Wirkstoffkombination verkapselt in Liposomen vorliegt neben unverkapselter Wirkstoffkombination.

4. Produkt nach Anspruch 1, dadurch gekennzeichnet, daß ein Teil der Wirkstoffkombination eingelagert in asymmetrischen lamellaren Aggregaten aus Fluorcarbonen und Phospholipiden vorliegt.

5. Produkt nach Anspruch 1, dadurch gekennzeichnet, daß die Wirkstoffkombination neben mit Sauerstoff beladenen asymmetrischen lamellaren Aggregaten aus Fluorcarbonen und Phospholipiden vorliegt.

6. Produkt nach Anspruch 1, dadurch gekennzeichnet, daß als Wirkstoff mit sphärischem TiO₂ oder SiO₂ modifiziertes Kaolin enthalten ist.

7. Produkt nach Anspruch 1, dadurch gekennzeichnet, daß als UV-Filter agglomerierte Substrate von TiO₂ und/oder ZnO vorliegen, die einen Gehalt an sphärischen und porösen SiO₂-Teilchen aufweisen, wobei die SiO₂-Teilchen eine Teilchengröße im Bereich von 0,05 *µ*m bis 1,5 *µ*m haben, und neben den SiO₂-Teilchen andere anorganische teilchenförmige Stoffe mit sphärischer Struktur vorliegen, wobei die sphärischen SiO₂-Teilchen mit den anderen anorganischen Stoffen definierte Agglomerate mit einer Teilchengröße im Bereich von 0,06 *µ*m bis 5 *µ*m bilden.

8. Produkt nach Anspruch 1, dadurch gekennzeichnet, daß die Konzentration der Wirkstoffkombination im Bereich von 0,1 bis 30 Gew-% liegt.

9. Produkt nach einem der Ansprüche 1 bis 8, dadurch gekennzeichnet, daß es Vitamin A enthält.

10. Verwendung des kosmetischen Produktes nach Anspruch 1 bis 9 in kosmetischen Masken, Cremes, Lotionen, Gelen, Haarpflegemitteln und Erzeugnissen der dekorativen Kosmetik.

## Revendications

1. Produit cosmétique pour la régénération et la stimulation des cellules de la peau, à base de constituants provenant de la mer, caractérisé par 0,01 à 50% en poids, ramené à la masse totale, d'une combinaison de principes actifs comprenant :
a) un produit d'un processus d'extraction enzymatique du plancton marin *Artemia salina*, le produit d'extraction se composant de nucléotides phosphorylés avec comme principal composant le tétraphosphate de diguanosine ;
b) du 1,4,5-triphosphate de D-myo-inositol ;
c) du glycane ;
le ratio a:b:c se situant dans la plage de 1 : 0,1 à 50 : 0,1 à 30 ;
et une teneur en autres principes actifs et véhicules cosmétiques de 99,99 à 50% en poids.

2. Produit selon la revendication 1, caractérisé en ce que le ratio a:b:c se situe dans la plage 1 : 0,5 à 10 : 0,5 à 8.

3. Produit selon la revendication 1, caractérisé en ce qu'une partie de la combinaison de principes actifs est présente encapsulée dans des liposomes en plus de combinaison de principes actifs non encapsulée.

4. Produit selon la revendication 1, caractérisé en ce qu'une partie de la combinaison de principes actifs est présente incluse dans des agrégats lamellaires asymétriques à base de fluorocarbones et de phospholipides.

5. Produit selon la revendication 1, caractérisé en ce que la combinaison de principes actifs est présente en plus d'agrégats lamellaires asymétriques à base de fluorocarbones et de phospholipides chargés avec de l'oxygène.

6. Produit selon la revendication 1, caractérisé en ce qu'il contient comme principe actif du kaolin modifié avec du TiO₂ ou SiO₂ sphérique.

7. Produit selon la revendication 1, caractérisé en ce qu'il contient en tant que filtres UV des substrats de TiO₂ et/ou ZnO aggloméré qui présentent une teneur en particules de SiO₂ sphériques et poreuses, les particules de SiO₂ ayant une granulométrie dans la plage de 0,05 µm à 1,5 µm, et, en plus des particules de SiO₂, d'autres substances inorganiques particulaires ayant une structure sphérique sont présentes, les particules de SiO₂ sphériques formant avec les autres substances inorganiques des agglomérats définis ayant une granulométrie dans la plage de 0,06 µm à 5 µm.

8. Produit selon la revendication 1, caractérisé en ce que la concentration de la combinaison de principes actifs se situe dans la plage de 0,1 à 30% en poids.

9. Produit selon l'une des revendications 1 à 8, caractérisé en ce qu'il contient de la vitamine A.

10. Utilisation du produit cosmétique selon les revendications 1 à 9 dans des masques cosmétiques, des crèmes, des lotions, des gels, des produits de soins capillaires et des produits de cosmétique décorative.

## Claims

1. A maritime-based cosmetic product for regeneration and stimulation of skin cells, comprising 0.01 to 50 wt.%, relative to the total mass, of an active ingredient combination made of
a) a product of an enzymatic extraction process of sea plankton Artemia salina, wherein the extraction product is comprised of phosphorylated nucleotides with the main component diguanosine-tetraphosphate;
b) D-myo-inosite-1,4,5-triphosphate;
c) glycan;
wherein the ratio a : b : c is in the range of 1 : 0.1-50 : 0.1-30;
and a content of further cosmetic active ingredients and carrier substances of 99.99 to 50 wt.%.

2. The product according to claim 1, wherein the ratio a : b : c is in the range of 1 : 0.5-10 : 0.5-8.

3. The product according to claim 1, wherein a portion of the active ingredient combination is present encapsulated in liposomes, in addition to active ingredient combination that is not encapsulated.

4. The product according to claim 1, wherein a portion of the active ingredient combination is contained in asymmetric lamellar aggregates of fluorocarbons and phospholipids.

5. The product according to claim 1, wherein the active ingredient combination is present in addition to asymmetric lamellar aggregates of fluorocarbons and phospholipids laden with oxygen.

6. The product according to claim 1, wherein as an active ingredient kaolin, modified with spherical TiO₂ or SiO₂, is present.

7. The product according to claim 1, wherein as a UV filter agglomerate substrates of TiO₂ and/or ZnO are present, having a contents of spherical and porous SiO₂ particles, wherein the SiO₂ particles have a particle size in the range of 0.05 *µ*m to 1.5 *µ*m, and, in addition to the SiO₂ particles, other inorganic particle-like substances with spherical structure are present, wherein the spherical SiO₂ particles form with the other inorganic substances defined agglomerates of a particle size in the range of 0.06 *µ*m to 5 *µ*m.

8. The product according to claim 1, wherein the concentration of the active ingredient combination is in the range of 0.1 to 30 wt.%.

9. The product according to one of the claims 1 through 8, characterized in that it contains vitamin A.

10. The use of the cosmetic product according to claim 1 through 9, in cosmetic masques, creams, lotions, gels, hair care products, and decorative cosmetic products.
